# EUROPEAN PATENT SPECIFICATION

(11) **EP 1 447 104 B1**
(45) Date of publication and mention of the grant of the patent: **07.10.2009**
(21) Application number: 04001417.7
(22) Date of filing: 23.01.2004
(51) Int. Cl.: A61L 24/00, A61L 27/46, A61L 27/24, A61F 2/28

(54) **Process to synthetize artificial bone tissue, and use thereof**
Verfahren zur Herstellung von künstlichem Knochengewebe und dessen Verwendung
Procédé de production d'un tissue osseux artificiel et son utilisation

(30) Priority: 05.02.2003 IT mi20030186
(43) Date of publication of application: 18.08.2004
(73) Proprietor: CONSIGLIO NAZIONALE DELLE RICERCHE, 00185 Roma (IT)
(72) Inventor: Tampieri, Anna, 48018 Faenza (RA) (IT); Celotti, Giancarlo, 48010 Faenza (RA) (IT); Roveri, Norberto, 40133 Bologna (IT); Landi, Elena, 40050 Dozza (BO) (IT)
(74) Representative: Bottero, Carlo

(56) References cited:
- DE-A- 19 930 335
- US-A- 5 231 169
- US-A1- 2002 183 855
- ITOH S ET AL: "Development of an artificial vertebral body using a novel biomaterial, hydroxyapatite/collagen composite" BIOMATERIALS, ELSEVIER SCIENCE PUBLISHERS BV., BARKING, GB, vol. 23, no. 19, October 2002 (2002-10), pages 3919-3926, XP004374384 ISSN: 0142-9612
- BIGI A ET AL: "Hydroxyapatite-gelatin films: a structural and mechanical characterization" BIOMATERIALS, ELSEVIER SCIENCE PUBLISHERS BV., BARKING, GB, vol. 19, no. 7-9, 5 April 1998 (1998-04-05), pages 739-744, XP004120842 ISSN: 0142-9612
- WANG R Z ET AL: "Synthesis of nanophase hydroxyapatite/collagen" JOURNAL OF MATERIALS SCIENCE LETTERS, CHAPMAN AND HALL LTD. LONDON, GB, vol. 14, no. 7, 1 April 1995 (1995-04-01), XP002131870 ISSN: 0261-8028
- ROVERI N ET AL: "Biologically inspired growth of hydroxyapatite nanocrystals inside self-assembled collagen fibers" COMPENDEX, 3 March 2003 (2003-03-03), XP002250940

## Description

It is known that natural bones are made of lamellar hydroxyapatite crystals grown in close contact with collagen fibrils. The properties of artificial bone tissue widely differ from those of natural bone tissue, said differences being mainly due to the absence of specific interactions between hydroxyapatite crystals and self-assembled protein components of natural hard tissues.

Collagen is about 30% by weight of total protein body and grants strength and structural stability to the various tissues, from skin to bones. More than 25 collagen sub-types have already been identified, the most known being type I, which is the main constituent of tendon and bone tissue. On the other hand, about 70% by weight of bone tissue is made of an impure inorganic phase, hydroxyapatite with a low crystallinity degree closely bonded in a regular way to collagen fibers. During the mineralization process of bone tissues, collagen is first synthesized, extruded from the cell and then self-assembled in the extra-cellular space before mineralization starts. That is why bone is the typical example of a mineralization process of "a mediated organic matrix". Electronic transmission micrography shows that bone crystals are basically prism-shaped. They are the smallest crystals having a biological formation, whose length and width changes depending on bone type. The smallest apatite crystals (2-5 nm thick and with a length of 35 nm and a width of 40 nm) have been found in calcified turkey tendon.

Today, the problem of bone lack has grown more important due to several factors such as more and more widespread dramatic events, cancers, osteoporosis, prosthesis revision surgery. It should be pointed out that the duration of prostheses in time is limited to about ten years and therefore, when the prosthesis has to be replaced, cavities have to be filled up with bone-similar tissue.

The main techniques for preparing bone-similar materials known until today are listed in the following.

Autogenous collections by taking from patients a bone portion, usually the iliac crest (cartilaginous bone area), which can reproduce and generate bone tissue. However, it is obvious that this method allows to fill up only small bone voids.

Preparation of bone-replacing material by taking hydroxyapatite from human and animal corpses and subsequent high-temperature treatment with acids so as to avoid immune responses. Said treatments, however; are not sufficient to ensure the elimination of certain viral forms, since the virus can crystallize and resist to acid treatments, also at high temperatures.

Preparation of hydroxyapatite with laboratory materials. However, this involves drawbacks connected to the fact that this hydroxyapatite does not have the same properties as natural hydroxyapatite formed through a mineralization process on collagen fibrils. In order to obtain organic protein hydroxyapatite composite materials, some protein matrixes having a different chemical composition and molecular structure can be prepared. To this purpose a biomimetic approach has been used for synthesizing calcium phosphates on various macromolecular matrixes, which act as templates and cause an oriented deposit of minerals. Chemical-physical properties of organic protein hydroxyapatite composite materials are strongly affected not only by chemical interactions between hydroxyapatite crystals and the protein matrix, but also by the structural organization of said matrix. In this case glutaraldehyde has been used as reticulating agent for organizing gelatin molecules; in other cases a method of titration and simultaneous co-precipitation with swine atelocollagen as starting material has been used.

Collagen, hydroxyapatite and mixtures thereof are well known. Conversely, no method for preparing a ceramic organic composite material having the same properties of human bone tissue is known.

Kikuchi M et al, Biomaterials 22/2001) synthesized a hydroxyapatite and collagen composite by a simultaneous titration coprecipitation method using ca(OH)₂, H₃PO₄ and porcine atelocollagen.

The present invention aims at preparing artificial bone tissue having the same properties as human bone tissue. Such aim is achieved by the present invention through a process of direct nucleation of an apatite phase on collagen fibrils, in which a solution of calcium salts promoting the formation of hydroxyapatite is reacted with collagen-similar natural polymers in acid suspension. A phosphoric component in aqueous solution is then added prop wise into the mixture of calcium salts in aqueous solution and collagen-similar natural polymers in acid suspension. The product thus obtained then undergoes lyophilization and/or filtration and drying.

The structure of the collagen-similar natural polymer transfers onto a molecular level the information enabling a chemical replacement of hydroxyapatite, just as it happens in the human body, i.e. substituants are let in, such as carbonate fractions and phosphate-type ion fractions present in the reaction mixture. The product obtained through said process has the same properties as human bone tissue thanks to the presence of said substituants in the structure of the composite material thus obtained.

Direct nucleation acts as a biological mechanism by forming hydroxyapatite nanocrystals grown parallel to the long axis of collagen fibrils, just as it happens in the human body, and results in an inorganic component with a low crystallinity degree, almost amorphous and therefore highly soluble. The crystallites thus obtained do now grow bigger and their size is of some nanometers. The nucleation of hydroxyapatite in collagen involves a carbonation of the inorganic phase, i.e. incorporation of CO₃²⁻ into hydroxyapatite reticule during the nucleation on B site in the same proportion as calcified natural bone tissue. Carbonation can further be assigned only to B position. The interaction of hydroxyapatite with collagen prevents carbonation in A position and is likely to block the access to -OH groups. This deviation from stoichiometry surprisingly increases the similarity with natural bone tissue, and thus not only the microstructure but also the composition of the obtained artificial bone tissue is just the same as the one of natural bone tissue. Carbonation in B position is advantageous with respect to carbonation in A position since, as a consequence, the bioactivity and biodegradability of the obtained artificial bone tissue are higher, which properties are essential for calcification since they enable a continuous dynamic exchange between the physiologic fluid and the cell using the ions for bone formation.

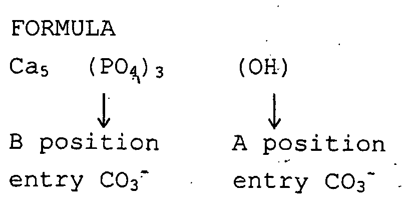

The present invention uses a biological approach for synthesizing hydroxyapatite nanocrystals on self-assembling collagen fibrils identical to natural bone nanocrystals, exploiting the ability of negatively charged collagen carboxylic groups to bind hydroxyapatite calcium ions; by following this approach it has been successfully shown that biological systems store and process information at molecular level. That is way molecules of collagen type I without telopeptides and free from glucosylated regions, which can self-aggregate into fibrils, without reticulating agents have been used as starting materials.

### Collagen preparation.

Collagen type I (taken from horse tendon) has been obtained on the market as a standard product. After purification collagen type I is dissolved in a calculated volume of acetic acid until a homogeneous suspension is obtained (1% collagen).

### Process according to the present invention.

According to the present invention calcium salts in aqueous solution promoting the formation of hydroxyapatite have been used, chosen from calcium hydroxide. Moreover, an acid suspension of collagen-similar natural polymer has been used, obtained by treatment of animal collagen taken from horse tendon, mouse tail, kangaroo tail, or of collagen-similar synthetic polymer or of chemically reticulated gelatins. The phosphate component used is chosen from phosphoric acid in aqueous solution. Calcium salts used in the present invention are in an amount of 1 to 4 g/l, the phosphate component is in an amount of 2 g/l to 4 g/l. More preferably, calcium salts used in the present invention are in an amount of 2 to 3 g/l, the phosphate component is in an amount of 3 to 4 g/l.

The direct nucleation step takes place at a pH value of 10 to 11 and a temperature preferably of 25 to 45°C, more preferably of 35 to 40°C.

A solution of phosphoric acid (1.17 g of H₃PO₄ in 200 cc of H₂O) and 50 g of collagen in acetic acid are added prop-wise into an aqueous solution of Ca(OH)₂ (1,47 g of Ca(OH)₂ in 300 cc of H₂O). The pH value during the process was of 10-11, the temperature was around 25°C, the prop-wise addition time of the phosphoric acid solution into the mixture of calcium salts in aqueous solution and collagen acid suspension was of 15 to 60 minutes and therefore dripping speed was preferably of 0.0133 1/minute to 0.0033 1/minute, more preferably 0.0100 to 0.0060 1/minute.

The obtained products, referred to with HA/Col_{nuc}., then underwent a lyophilization or drying step.

Then the composite products have been characterized by crystallographic analysis with an X-ray diffractometer (Rigaku Miniflex); for orientation analysis some diffraction graphs have been photographically recorded with a sample-film distance of 70 mm using a flat camera (always with a Cu-Kα radiation).

Then a thermogravimetric analysis (Polymer STA 1660) has been made using an alumina crucible in air and a heating speed of 10°C/min.

Analysis of composite materials.

The composite materials prepared with the method according to the present invention, by direct nucleation of hydroxyapatite in collagen fibrils, had a nominal composition hydroxyapatite/collagen of 80/20 and 60/40 by weight. However, the thermogravimetric analysis has shown that the actual composition was 65/35 and 50/50, respectively. This difference is due to reaction yield which does not reach 100%. The water content in the compound is very similar to pure collagen (10%). This analysis has been successfully used to obtain information on the modifications of structural relations between collagen fibrils and inorganic phase as a function of mineral content. Such analysis was previously executed on the flexor tendon of a turkey leg, which is used as a model in the calcification process.

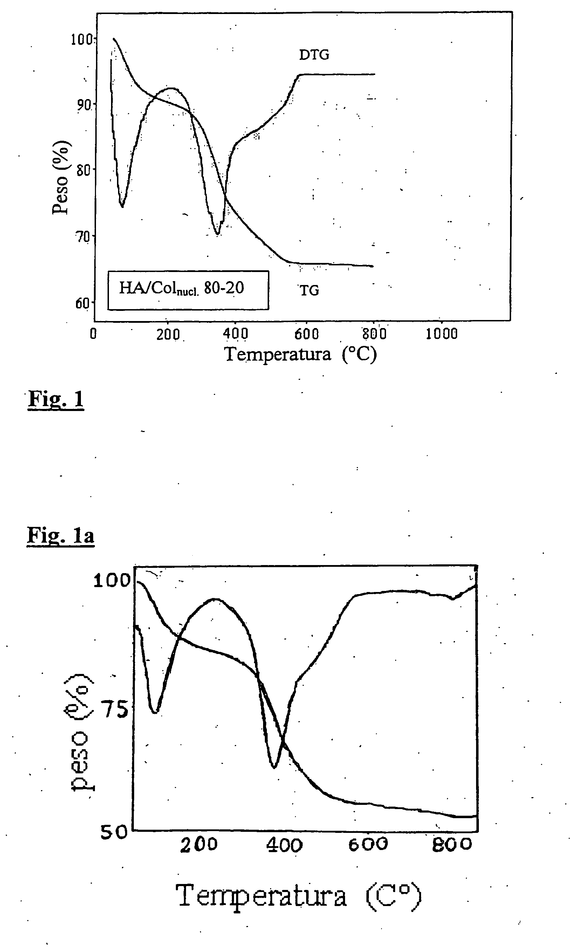

As a matter of fact, TG-DTG graphs (thermogravimetry-digital thermogravimetry) of Fig. 1 are different from those of hydroxyapatite/collagen compounds, which shown an interaction of organic phase with collagen fibers. This kind of interaction can be compared with the calcification process occurring in nature. Fig. 1a shows TG-DTG graphs concerning a calcified turkey leg, used as a theoretical bone model. The close similarity is evident since in both cases DTG peak at 450-500°C tends to disappear forming a "widened shoulder". It can be assumed that when a direct nucleation of hydroxyapatite on collagen occurs, the composite material behaves like naturally calcified tissue.

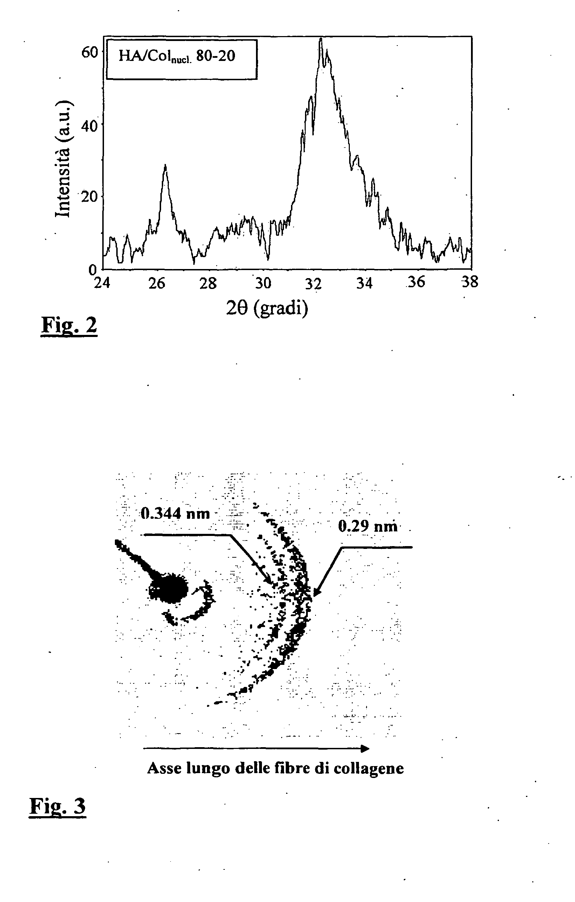

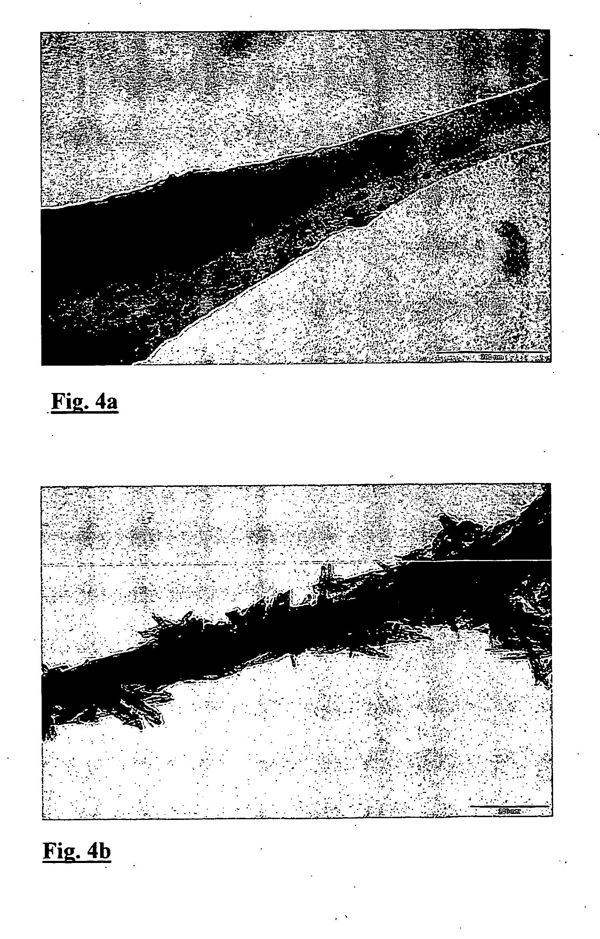

The XRD diffractogram of Fig. 2 shows a typical graph of hydroxyapatite with a very low crystallinity, the size of crystallites identified on axis c being of about 12-15 nm. Nucleation occurs in accordance with the process typical of natural bone, in which the nano-size of crystallites is responsible for the widening of reflection in the graph. Moreover, since the reaction has been carried out at about 25°C, nanocrystals of monocrystalline hydroxyapatite can be obtained with a high degree of certainty. Such nanocrystals grow within collagen fibers with their axis c oriented preferably parallel to fiber orientation direction. As a matter of fact, the X-ray wide angle diffraction graph is obtained starting from a sample made of some calcified fibrils. Fig. 3 shows the (002) reflection of hydroxyapatite at 0.344 nm preferably oriented parallel to the orientation direction typical of molecular collagen distanced at 0.29 nm. TEM micrography in Figs. 4a and 4b shows nano-nuclei formed within collagen fibrils growing parallel to said fibrils. In particular, Figure 4a shows an initial stage of nucleation, whereas Figure 4b shows an advanced stage of nucleus growth.

Such analytical techniques have shown strong interactions between the two hydroxyapatite components and a full analogy of the composite material with calcified natural tissue.

Moreover, the analysis with an optical microscope shows the different crystalline morphology of the composite material hydroxyapatite/collagen obtained by direct nucleation and then lyophilized from the air-dried one. The lyophilized composite materials show a three-dimensional network characterized by a very wide pore distribution resembling cotton and wool. When the sample is air-dried on a filter, a bi-dimensional network forms and its structure resembles a gauze.

The artificial bone tissue according to the present invention can be obtained with different amounts of hydroxyapatite/collagen and various formulations and applies to several chemical uses. Such artificial bone tissue can be used as prosthesis, bone-replacing or bone-filling material, as rebuilding membrane or haemostatic tissue in orthopedics, dentistry, maxillo-facial surgery.

### Brief description of figures.

Figure 1: TG-DTG graphs (thermogravimetry - digital thermogravimetry) of composite materials obtained by direct nucleation.
Figure 1a: TG-DTG graphs (thermogravimetry - digital thermogravimetry) of calcified turkey tendon used as theoretical bone model.
Figure 2: X-ray diffractogram of low crystallinity hydroxyapatite.
Figure 3: Diffraction measurements with flat camera show the orientation of axis C of the hydroxyapatite crystalline fiber in the same direction as the long axis of the fiber.
Figure 4a: TEM micrography of nano-nuclei formed in situ, in an initial stage of development.
Figure 4b: TEM micrography of nano-nuclei formed in situ, in an advanced stage of development.

## Claims

1. Process to synthesize an artificial bone tissue comprising:
directly nucleating nanocrystals of a monocrystalline hydroxyapatite within collagen fibers by adding drop-wise an aqueous solution of phosphoric acid and collagen in acetic acid into an aqueous solution of calcium hydroxide;
lyophilizing or drying the so obtained product.

2. The process according to claim 1, wherein the rate of drop-wise addition is 0.0133 1/minute to 0.0033 1/minute.

3. The process according to claim 2, wherein the rate of drop-wise addition is 0.01 to 0.006 1/minute.

4. The process according to any one of claims from 1 to 3, wherein the direct nucleation step takes place at a pH value of 10-11.

5. The process according to any one of claims from 1 to 4, wherein the direct nucleation step is carried out at around 25°C.

6. Artificial bone tissue obtained according to any one of claims from 1 to 5 for use as prosthesis, bone-replacing, bone-filling material, as rebuilding membrane, homeostatic and haemostatic tissue and in dental applications.

## Patentansprüche

1. Verfahren zur Herstellung eines künstlichen Knochengewebes umfassend die folgenden Schritte:
direkte Nukleation von Nanokristallen eines monokristallinen Hydroxyapatits innerhalb Kollagenfasern durch Zutropfen einer wässrigen Lösung von Phosphorsäure und Kollagen in Essigsäure in eine wässrige Lösung von Calciumhydroxid;
Gefriertrocknung oder Trocknung des so erhaltenen Produktes.

2. Verfahren nach Anspruch 1, worin die Förderrate des Zutropfens 0,0133 1/Minute bis 0,0033 1/Minute beträgt.

3. Verfahren nach Anspruch 2, worin die Förderrate des Zutropfens 0,01 bis 0,006 1/Minute beträgt.

4. Verfahren nach irgendeinem der Ansprüche 1 bis 3, worin der Schritt von direkter Nukleation bei einem pH-Wert von 10-11 erfolgt.

5. Verfahren nach irgendeinem der Ansprüche 1 bis 4, worin der Schritt von direkter Nukleation bei ca. 25°C durchgeführt wird.

6. Künstliches Knochengewebe, das nach irgendeinem der Ansprüche 1 bis 5 erhalten wird, zur Verwendung als Prothese-, Knochenersatz-, Knochenfüllmaterial, als Wiederherstellungsmembrane, homöostatisches und hämostatisches Gewebe und in zahnärztlichen Anwendungen.

## Revendications

1. Procédé de production d'un tissu osseux artificiel comprenant les étapes suivantes:
nucléation directe de nanocristaux d'une hydroxyapatite monocristalline dans des fibres de collagène par addition goutte à goutte d'une solution aqueuse d'acide phosphorique et collagène en acide acétique dans une solution aqueuse d'hydroxyde de calcium;
lyophilisation ou séchage du produit ainsi obtenu.

2. Procédé selon la revendication 1, où le débit de l'addition goutte à goutte est de 0,0133 1/minute à 0,0033 1/minute.

3. Procédé selon la revendication 2, où le débit de l'addition goutte à goutte est de 0,01 à 0,006 1/minute.

4. Procédé selon une quelconque des revendications 1 à 3, où l'étape de nucléation directe a lieu avec une valeur de pH de 10-11.

5. Procédé selon une quelconque des revendications 1 à 4, où l'étape de nucléation directe est effectuée à 25°C environ.

6. Tissu osseux artificiel obtenu selon une quelconque des revendications 1 à 5, pour l'emploi en tant que matériau pour prothèse, remplacement d'os, remplissage d'os, en tant que membrane de reconstruction, tissu homéostatique et hémostatique et dans des applications dentaires.
